# EUROPÄISCHE PATENTSCHRIFT

(11) **EP 3 027 167 B1**
(45) Veröffentlichungstag und Bekanntmachung des Hinweises auf die Patenterteilung: **09.08.2017**
(21) Anmeldenummer: 14747818.4
(22) Anmeldetag: 26.06.2014
(51) Int. Cl.: A61K 8/02, A61K 8/25, A61K 8/92, A61K 8/34, A61K 8/58, A61K 8/891, A61Q 15/00, A61K 8/26, A61K 8/41, A61K 8/89

(54) **WASSERFREIE ÖL/VERDICKUNGSMISCHUNGEN ALS GRUNDLAGE FÜR KOSMETISCHE SOFT SOLID- UND/ODER STIFTPRÄPARATE**
WATER FREE OIL/THICKENER-MIXTURES AS BASE FOR COSMETIC SOFT, SOLID- OR STICKPREPARATIONS
MELANGE HUILE/ÉPAISSISSANT SANS EAU COMME FONDATION POUR PRÉPARATIONS COSMÉTIQUES DOUCES, SOLIDES OU BÂTONS.

(30) Priorität: 30.07.2013 DE 102013214938
(43) Veröffentlichungstag der Anmeldung: 08.06.2016
(73) Patentinhaber: Henkel AG & Co. KGaA, 40589 Düsseldorf (DE)
(72) Erfinder: DÖRING, Thomas, 41540 Dormagen (DE)
(86) Internationale Anmeldenummer: PCT/DE2014/200284
(87) Internationale Veröffentlichungsnummer: WO 2015/014354

(56) Entgegenhaltungen:
- EP-A2- 0 117 070
- WO-A1-97/17942
- US-A- 4 526 780

## Beschreibung

Die vorliegende Erfindung liegt auf dem Gebiet der Kosmetik und betrifft wasserfreie Zusammensetzungen, die neben einem Öl eine spezifische Kombination von Verdickungsmitteln enthalten. Die Erfindung betrifft weiterhin die Verwendung von Öl/Verdickungsmittelmischungen als Grundlage für kosmetische soft solid- und/oder Stiftpräparate.

Kosmetische Stiftpräparate wie beispielsweise Lippen- oder Deodorantstifte sind seit langem bekannt und werden in den verschiedensten Bereichen der Körperpflege von vielen Verbrauchern regelmäßig angewendet.
Sie können jedoch den Nachteil aufweisen, dass sie bei der Anwendung auf der Haut als zu hart empfunden werden. Weiterhin problematisch sind mitunter die Dosierung der Produkte sowie die unerwünschte Neigung einiger Stiftpräparate zur Bildung von Rückständen auf der Anwendungsoberfläche.
Aus diesen Gründen wurden die so genannten weichen Feststoffe ("soft solids") als weitere Anwendungsform entwickelt, die - gegenüber Stiftpräparaten - von vielen Verbrauchern als angenehmer empfunden werden.

Unter "soft solids" werden viskose Zusammensetzungen verstanden, die meist eine cremige Textur aufweisen und üblicherweise vor der Anwendung durch eine oder mehrere Öffnungen einer Abgabevorrichtung des Applikators herausgedrückt werden. Bei diesem Vorgang wird ein Druck auf die Zusammensetzung ausgeübt, unter dem die Rezeptur oftmals instabil wird und dabei einen seiner flüssigen Bestandteile abscheidet. Dieses Phänomen wird Synerese genannt und ist meist bei soft solids mit hohem Ölgehalt zu beobachten.
Problematisch und unerwünscht sind bereits Produkte, die bei 50°C eine Synerese von >8% aufweisen.

Wasserfreie soft solids sowie wasserfreie Stiftpräparate enthalten meist Suspensionen eines (oder mehrerer) Hilfs- und/oder Wirkstoffs(e) in einem unpolaren Öl, welches zur Verhinderung der Sedimentation des(r) Hilfs- und/oder Wirkstoffs(e) mindestens ein Verdickungsmittel enthält. Es wurde gefunden, dass eine Vielzahl im Handel bekannter Verdickungsmittel(systeme) die unerwünschte Synerese begünstigen, insbesondere wenn die Zubereitungen, wie zuvor beschrieben, durch Anwendung von Druck aus dem Applikator gedrückt werden (soft solids).

Aufgabe der vorliegenden Anmeldung war es daher, eine kosmetische Zusammensetzung in Form einer wasserfreien Zusammensetzung als soft solid bereitzustellen, die keine oder zumindest eine verminderte Synerese aufweist. Darüber hinaus soll die Zusammensetzung die für eine gute Verteilbarkeit auf der Haut notwendige Härte und Textur aufweisen.
Unter notwendiger Härte wird bevorzugt eine Härte von 0,015 bis 0,045 mN, mehr bevorzugt von 0,016 bis 0,044 mN, besonders bevorzugt von 0,017 bis 0,043 mN und insbesondere von 0,018 bis 0,042 mN verstanden.
Unter verminderter Synerese wird bevorzugt eine Synerese von < 7,5%, mehr bevorzugt
< 7,25%, besonders bevorzugt < 7% und insbesondere < 6,75% verstanden.
Schließlich sollte die Zusammensetzung nach der Anwendung kein klebriges Gefühl auf der Haut und keine Rückstände auf den Textilien hinterlassen.

Es wurde gefunden, dass die gestellte Aufgabe durch folgenden ersten Erfindungsgegenstand gelöst wird: Wasserfreie Zusammensetzung, enthaltend:
- mindestens ein Öl, und
- eine Kombination von Verdickungsmitteln, enthaltend
   a) mindestens ein Siliconelastomer,
   b) mindestens einen geradkettigen oder verzweigten, gesättigten oder ungesättigten Alkohol mit mehr als 18 Kohlenstoffatomen, und
   c) mindestens eine mit quaternären Ammoniumverbindungen modifizierte Tonmineral-Mischung.

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Der Begriff "wasserfrei" wird erfindungsgemäß so verstanden, dass die Zusammensetzungen 0 bis maximal 3 Gew.-%, bevorzugt 0 bis maximal 2 Gew.-%, freies Wasser enthalten, bezogen auf die gesamte Zusammensetzung. Der Gehalt an Kristallwasser, Hydratationswasser oder ähnlich molekular gebundenem Wasser, der in den eingesetzten Bestandteilen, insbesondere in gegebenenfalls enthaltenen schweißhemmenden Wirkstoffen, enthalten sein kann, stellt im Sinne der vorliegenden Anmeldung kein freies Wasser dar.

Die erfindungsgemäßen wasserfreien Zusammensetzungen eignen sich prinzipiell für alle kosmetischen Applikationsformen, in denen soft-solids und/oder Stiftpräparate üblicherweise angewendet werden (beispielsweise Lippenstifte, soft-solid-Lippenpflegeformulierungen, Fettstifte- und/oder soft-solid-Formulierungen für die Haut, Antitranspirantien etc.).

In einer bevorzugten Ausführungsform werden die erfindungsgemäßen wasserfreien Zusammensetzungen jedoch als soft solid konfektioniert und insbesondere als Antitranspirant verwendet.

Erfindungsgemäße wasserfreie Zusammensetzungen, die als Antitranspirant verwendet werden, enthalten zusätzlich mindestens einen Antitranspirant-Wirkstoff (der auch als schweißhemmender Wirkstoff bezeichnet wird).
Der Antitranspirant-Wirkstoff wird in den erfindungsgemäßen wasserfreien Zusammensetzungen, die als Antitranspirant verwendet werden, bevorzugt in einer Menge von 3 bis 35 Gew.-%, mehr bevorzugt von 5 bis 30 Gew.-% und besonders bevorzugt von 10 bis 25 Gew.-% eingesetzt, wobei sich die Mengen auf das Gesamtgewicht der kristallwasserfreien Aktivsubstanz (USP) in der Gesamtzusammensetzung beziehen.

Bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze.
Unter Wasserlöslichkeit wird erfindungsgemäß eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.
Besonders bevorzugte Antitranspirant-Wirkstoffe sind ausgewählt aus Aluminiumchlorhydrat, insbesondere Aluminiumchlorhydrat der allgemeinen Formel [Al₂(OH)₅Cl x 1-6 H₂O]ₙ, bevorzugt [Al₂(OH)₅Cl x 2-3 H₂O]ₙ, das in nicht-aktivierter oder in aktivierter (depolymerisierter) Form vorliegen kann, sowie Aluminiumchlorhydrat der allgemeinen Formel [Al₂(OH)₄Cl₂ x 1-6 H₂O]ₙ, bevorzugt [Al₂(OH)₄Cl₂ x 2-3 H₂O]ₙ, das in nicht-aktivierter oder in aktivierter (depolymerisierter) Form vorliegen kann.
Die Herstellung bevorzugter Antitranspirant-Wirkstoffe ist beispielsweise in US 3887692, US 3904741, US 4359456, GB 2048229 und GB 1347950 offenbart.
Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ x 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat). Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- oder Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(AI+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt. Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt. Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, wie sie beispielsweise in US 6902723 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(AI+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt. Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein AI:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein AI:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen. Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart. Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen.

Bevorzugte Aluminiumsalze und Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (AI+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:CI = 0,98 - 0,9) sowie sehr bevorzugt m = 1,04 - 1,11 (entsprechend M:CI = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.
Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders

bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrat (Al:Zr = 2-6; M:CI = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

Die Antitranspirant-Wirkstoffe können als nicht-wässrige Lösungen oder als glycolische Solubilisate eingesetzt werden.

In einer besonders bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine oder Superultrafine von Reheis, Microdry 323 von Summit, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Besonders bevorzugt sind auch aktivierte Aluminiumchlorohydrate, die unter den Bezeichnungen Reach^{®} 101 und Reach^{®} 103 , AACH- 7171 von Reheis oder Summit erhältlich sind. Auch die Verwendung von Aluminium-Zirkonium-Tetrachlorohydrex-Glycin-Komplexen, die beispielsweise von Reheis unter der Bezeichnung Rezal^{®} 36 GP von Reheis oder AZG-364 oder 369 von Summit, in aktivierter Qualität, als Reach^{®} 908, als Pulver im Handel sind, kann erfindungsgemäß besonders bevorzugt sein.

Insbesondere bevorzugt sind Aluminium-Zirkoniumpentachlorohydex-Glycin-Komplexe (AAZG-3108 oder AAZG-3110 von Summit), Aluminium-Zirkoniumtetrachlorohydex-Glycin-Komplexe ( AAZG-3111 von Summit) und/oder Aluminium-Zirkoniumoctachlorohydex-Glycin-Komplexe ( AAZG-3109, AAZG-531 oder AAZG-531 D von Summit oder Zirkonal® AP3G von BK Giulini).

Die erfindungsgemäße Zusammensetzung enthält zwingend mindestens ein Öl.

Das mindestens eine Öl wird in den erfindungsgemäßen Zusammensetzungen bevorzugt in einer Menge von 20 bis 85 Gew.-%, mehr bevorzugt von 30 bis 80 Gew.-%, besonders bevorzugt von 40 bis 75 Gew.-% und ganz besonders bevorzugt von 50 bis 70 Gew.-% eingesetzt.

Unter einem Öl ist erfindungsgemäß ein flüssiger Stoff zu verstehen, der bei Normalbedingungen in bidestilliertem Wasser zu weniger als 1 Gew.-% mischbar ist.

Besonders bevorzugt enthält die erfindungsgemäße Zusammensetzung als Öl mindestens ein flüchtiges Öl. Dabei ist es wiederum bevorzugt, wenn die flüchtigen Öle bezogen auf das Gewicht der Zusammensetzung in einer Gesamtmenge von 30 bis 80 Gew.-%, besonders bevorzugt von 40 bis 75 Gew.-%, ganz besonders bevorzugt von 45 bis 70 Gew.-%, in der erfindungsgemäßen Zusammensetzung enthalten sind.

Als flüchtiges Öl werden erfindungsgemäß Öle verstanden, die bei 293,15 K einen Dampfdruck von 0,01 kPa oder mehr aufweisen.

Erfindungsgemäß bevorzugte Öle sind ausgewählt aus Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen.

Besonders bevorzugte flüchtige Öle sind flüchtige Silikonöle und flüchtige Nichtsilikonöle. Flüchtige Siliconöle, die cyclisch sein können, wie z. B. Octamethylcyclotetrasiloxan, Decamethylcyclopentasiloxan sowie Mischungen hiervon, wie sie z. B. in den Handelsprodukten DC 244, 245, 344 und 345 von Dow Corning enthalten sind, sind erfindungsgemäß besonders bevorzugt. Ebenfalls besonders bevorzugt sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind.

Flüchtige Siliconöle sind erfindungsgemäß hervorragend geeignet, da sie der erfindungsgemäßen Zusammensetzung ein angenehmes Hautgefühl und eine geringe Kleideranschmutzung verleihen. Erfindungsgemäß besonders bevorzugte Zusammensetzungen sind daher durch einen Gehalt an mindestens einem flüchtigen Siliconöl gekennzeichnet. Dabei ist es wiederum bevorzugt, wenn die flüchtigen Silikonöle bezogen auf das Gewicht der Zusammensetzung in einer Gesamtmenge von 20 bis 80 Gew.-%, insbesondere von 30 bis 80 Gew.-%, besonders bevorzugt von 40 bis 75 Gew.-%, ganz besonders bevorzugt von 45 bis 70 Gew.-%, in der erfindungsgemäßen Zusammensetzung enthalten sind.

Neben oder anstelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Iso-paraffinen, insbesondere aus Isodecan, Isododecan, Isotetradecan und Isohexadecan sowie Mischungen hiervon. Insbesondere eignet sich C10-13 Isoparaffin (z.B. Handelsprodukt Pioner 2094 von Hansen & Rosenthal).

Auch dieses mindestens eine flüchtige Nichtsiliconöl ist bevorzugt in einer Gesamtmenge von von 20 bis 80 Gew.-%, besonders bevorzugt von 30 bis 80 Gew.-%, ganz besonders bevorzugt von 40 bis 75 Gew.-%, jeweils bezogen auf das Gesamtgewicht der Zusammensetzung, enthalten.

Aufgrund des Hautgefühls und der Stabilität der resultierenden Zusammensetzungen sind Siliconöle als flüchtiges Öl gegenüber Isoparaffinen besonders bevorzugt.

Neben den vorgenannten, üblicherweise als flüchtigen Siliconölen bezeichneten Substanzen sowie neben den vorgenannten flüchtigen Nichtsiliconölen können die erfindungsgemäßen Zusammensetzungen zusätzlich mindestens ein nichtflüchtiges Öl, ausgewählt aus nichtflüchtigen Siliconölen und nichtflüchtigen Nichtsiliconölen, enthalten.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Baysilon^{®} 350 M (mit einer kinematischen Viskosität (25°C) von etwa 350 cSt.

Erfindungsgemäß ebenfalls bevorzugte nichtflüchtige Siliconöle sind ausgewählt aus Siliconen der Formel (Sil-1), wobei x ausgewählt ist aus ganzen Zahlen von 1 - 20, bevorzugt 1 - 3.

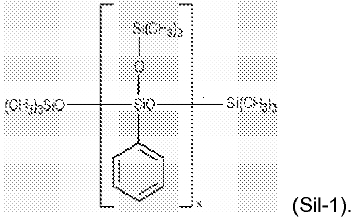

Ein bevorzugtes Siliconöl der Formel (Sil-1) ist erhältlich unter der INCI-Bezeichnung Phenyl Trimethicone.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis) gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD. Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Düsopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Düsooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Estern der linearen oder verzweigten gesättigten oder nichtflüchtige Nichtsiliconöle, Ester ungesättigter Alkanole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristyl-ether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Weitere erfindungsgemäß besonders bevorzugte nichtflüchtige Nichtsiliconöle sind ausgewählt aus den C₈-C₂₂-Alkanolestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß besonders bevorzugte nichtflüchtige Nichtsiliconöle sind ausgewählt aus den symmetrischen, unsymmetrischen oder cyclischen Estern der Kohlensäure mit Alkanolen, z. B. Glycerincarbonat, Dicaprylylcarbonat (Cetiol^{®} CC) oder die Ester gemäß der Lehre der DE 19756454 A1. Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es ist erfindungsgemäß bevorzugt, wenn die erfindungsgemäßen Zusammensetzungen bezogen auf das Gesamtgewicht der Zusammensetzung die nichtflüchtigen Öle in einer Gesamtmenge von 0 bis 20 Gew.-%, insbesondere von 0 bis 10 Gew.-% enthalten.

Es wurde gefunden, dass sich die erfindungsgemäßen wasserfreien Zusammensetzungen besonders gut und stabil verdicken lassen, wenn sie als Verdickungsmittelsystem eine Mischung spezifischer lipophiler Verdickungsmittel enthalten.

Die erfindungsgemäßen Zusammensetzungen enthalten daher zwingend eine Kombination der folgenden lipophilen Verdickungsmittel:
- mindestens ein Siliconelastomer,
- mindestens einen geradkettigen oder verzweigten, gesättigten oder ungesättigten Alkohol mit mehr als 18 Kohlenstoffatomen, und
- mindestens eine mit quaternären Ammoniumgruppen modifizierte Tonmineral-Mischung.

Unter erfindungsgemäß geeigneten Siliconelastomeren sind bevorzugt Verbindungen zu verstehen, die erhältlich sind durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicongebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.

Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040 Silicone Elastomer Blend (ein Cyclomethicone (and) Dimethicone Crosspolymer von Dow Corning; Siliconelastomergehalt 12 - 13 Gew.-%), SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie, insbesondere Gransil SR-CYC (Cyclomethicone and Stearyl-Vinyl/hydromethylsiloxane Copolymer), Gransil^{®} RPS Gel (INCI-Bezeichnung: Cyclopentasiloxane and Polysilicone-11), Gransil^{®} GCM-4 (INCI-Bezeichnung: Cyclotetrasiloxane and Polysilicone-11), Gransil^{®}GCM-5 (INCI-Bezeichnung: Cyclopentasiloxane and Polysilicone-11), Gransil^{®} RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil^{®} IDS (INCI-Bezeichnung: Isododecane (and) Cyclotetrasiloxane (and) Polysilicone-11), Gransil^{®}PC-12 (INCI-Bezeichnung: Isododecane (and) Polysilicone-11), Gransil^{®}IDS-5 (INCI-Bezeichnung: Isododecane (and) Cyclopentasiloxane (and) Polysilicone-11), Gransil^{®}APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil^{®}DMCM-5 (INCI-Bezeichnung: Dimethicone and Cyclopentasiloxane and Polysilicone-11), Gransil^{®}DMG-6 mit Dimethicone (6 cSt) (INCI-Bezeichnung: Dimethicone and Polysilicone-11), Gransil^{®}DMG-20 mit Dimethicone (20 cSt) (INCI-Bezeichnung: Dimethicone and Polysilicone-11), Gransil^{®} AM-8 Gel (INCI-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCI-Bezeichnung: Dimethicone and Isododecane and Polysilicone-11), Gransil^{®} PM (INCI-Bezeichnung: Phenyl Trimethicone and Polysilicone-11), Gransil^{®} ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} MLB (INCI-Bezeichnung: Cyclopentasiloxane and Polysilicone-11 and Beeswax), Gransil^{®} PS (INCI-Bezeichnung: Cyclotetrasiloxane and Polysilicone-11 and Petrolatum), Gransil^{®} PS-5 (INCI-Bezeichnung: Cyclopentasiloxane and Polysilicone-11 and Petrolatum), Gransil^{®} DMG-20 P mit Dimethicone (20 cSt) und Petrolatum (INCI-Bezeichnung: Dimethicone and Polysilicone-11 and Petrolatum), Gransil^{®} RJO (INCI-Bezeichnung: Cyclopentasiloxane and Polysilicone-11 and Jojoba Oil), Gransil^{®} LANO (INCI-Bezeichnung: Cyclopentasiloxane and Polysilicone-11 and Lanolin), Gransil^{®} OHS-5 (INCI-Bezeichnung: Cyclopentasiloxane and Polysilicone-11 and Octyl Hydroxystearate) und Gransil^{®} DML (INCI-Bezeichnung: Dimethicone (and) Neopentyl Glycol Diheptanoate (and) Polysilicone-11).

Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2,75 Gew.-%, besonders bevorzugt 0,15 - 2,5 Gew.-%, außerordentlich bevorzugt 0,2 - 2 Gew.-%, jeweils bezogen auf das Gesamtgewicht der erfindungsgemäßen Zusammensetzung, enthalten.

Die erfindungsgemäße Zusammensetzung enthält als zweiten Bestandteil der Verdickungsmittelmischung mindestens einen geradkettigen oder verzweigten, gesättigten oder ungesättigten Alkohol mit mehr als 18 Kohlenstoffatomen.
Der mindestens eine Alkohol wird in den erfindungsgemäßen Zusammensetzungen bevorzugt in einer Menge von 0,5 bis 10 Gew.-%, mehr bevorzugt von 1 bis 8 Gew.-%, besonders bevorzugt von 1,5 bis 7 Gew.-% und insbesondere bevorzugt von 2 bis 6 Gew.-% eingesetzt, wobei sich die Mengenangaben auf das Gesamtgewicht der erfindungsgemäßen Zusammensetzungen beziehen.

Es wurde gefunden, dass eine optimale Härte der soft solids und/oder der Stiftpräparate erreicht werden kann, wenn geradkettige oder verzweigte, gesättigte oder ungesättigte Alkohole (oder Alkoholmischungen) in den erfindungsgemäßen Zusammensetzungen verwendet werden, die bevorzugt einen Schmelzpunkt im Bereich von 75 bis 110°C, mehr bevorzugt von 80 bis 105°C und besonders bevorzugt von 85 bis 100°C aufweisen.
In einer bevorzugten Ausführungsform enthalten die erfindungsgemäßen Zusammensetzungen geradkettige oder verzweigte, gesättigte oder ungesättigte Alkohole mit mehr als 20 und weniger als 60 Kohlenstatomen. Besonders bevorzugt sind geradkettige, gesättigte Alkohole mit durchschnittlich 20 bis 40, bevorzugt 25 bis 35 und insbesondere bevorzugt mit durchschnittlich 30 Kohlenstoffatomen. Solche Alkohole sind im Handel erhältlich, beispielsweise unter der Handelsbezeichnung Performacol^{®} 425 von der Firma Baker Petrolite.

Als dritten zwingenden Bestandteil der Verdickungsmittelmischung enthalten die erfindungsgemäßen Zusammensetzungen mindestens eine mit quaternären Ammoniumgruppen modifizierte Tonmineral-Mischung.
Es wurde gefunden, dass der Zusatz der Tonmineralmischung die Viskositätsstabilität der erfindungsgemäßen Zusammensetzungen erhöht.
Die mit quaternären Ammoniumgruppen modifizierte Tonmineral-Mischung wird in den erfindungsgemäßen Zusammensetzungen - bezogen auf deren Gesamtgewicht - bevorzugt in einer Menge von 0,1 bis 5 Gew.-%, mehr bevorzugt von 0,2 bis 4 Gew.-%, besonders bevorzugt von 0,3 bis 3 Gew.-% und insbesondere bevorzugt von 0,5 bis 2,5 Gew.-% eingesetzt.

Bevorzugte Tonmineral-Mischungen im Sinne der vorliegenden Erfindung enthalten
i) mindestens ein Tonmineral, ausgewählt aus Sepiolith und/oder Palygorskit, und
ii) mindestens ein Tonmineral vom Smektit-Typ.
Diese Tonmineral-Mischungen werden mit quaternären Ammoniumverbindungen behandelt.

Es wurde gefunden, dass diese speziellen, mit quaternären Ammoniumgruppen modifizierten Tonmineralmischungen, herkömmlichen, mit quaternären Ammoniumgruppen modifizierten Tonmineralien (wie beispielsweise Distearyldimonium Hectorite) überlegen sind, denn sie erfordern nicht die zusätzliche Anwesenheit eines polaren Aktivators (wie beispielsweise Ethanol oder Propoylencarbonat), der in wasserfreie Zusammensetzungen nicht oder nur unter erheblichen Schwierigkeiten eingearbeitet und darin stabilisiert werden könnte.

Tonminerale vom Typ i) weisen üblicherweise eine stabförmige Struktur auf, während Tonminerale vom Typ ii) üblicherweise eine plättchenförmige Struktur aufweisen. Durch die Mischung der Tonmineraltypen i) und ii) werden nicht-einheitliche Strukturen erzielt, welche die Bildung von Zwischenräumen ermöglichen, in denen Wirkstoffe hervorragend suspendiert werden können. Weiterhin von Vorteil ist, dass die Re-Dispersion der Tonmineral-Mischung durch die nicht-einheitlichen Strukturen verhindert oder minimiert wird, wodurch die Viskositätsstabilität der erfindungsgemäßen Zusammensetzungen weiterhin gesteigert werden kann.
Durch die Modifikation der Tonmineral-Mischung mit quaternären Ammoniumgruppen werden abstoßende, sterische Hindernisse erzeugt, die zudem die Dispersion der Tonmineral-Mischung unterstützen.

Sepiolith (bekannt als Meerschaum; chemische Formel Mg₄Si₆O₁₅(OH)₂x6H₂O) und Palygorskit (bekannt als Bergleder; chemische Formel (Mg, Al)₂Si₄O₁₀(OH)x4H₂O) sind Papyrus-ähnliche oder faserige Magnesiumsilicate, die zu den Schichtsilicaten gezählt werden. Sie unterscheiden sich jedoch von anderen Schicksilikaten durch das Fehlen kontinuierlicher octahedraler Lagen.
Besonders bevorzugt wird als Tonmineral i) der Tonmineralmischung Sepiolith verwendet.

Erfindungsgemäß geeignete Tonmineralien vom Smektit-Typ umfassen Tonmineralien, welche di- oder tri-octahedrale Strukturen enthalten und plättchenförmige Strukturen aufweisen.
Zu den dioctahedralen Smektiten zählen Montmorillonit, Beidellit und Nontronit; zu den trioctaherdralen Smektiten zählen Saponit, Hectorit, Stevensit und Sauconit.
Synthetische hergestellte Smektite sind ebenfalls erfindungsgemäß geeignet.
In einer bevorzugten Ausführungsform sind die Tonmineralien vom Smektit-Typ (ii)) daher ausgewählt aus Hectorit, Montmorillonit, Bentonit, Beidellit, Saponit, Stevensit, Nontronit und/oder Sauconit.
In einer besonders bevorzugten Ausführungsform sind die Tonmineralien vom Smektit-Typ (ii)) ausgewählt aus Hectorit, Montmorillonit und/oder Bentonit, insbesondere bevorzugt ist Montmorillonit.

In einer weiteren bevorzugten Ausführungsform werden die Tonminerale vom Typ i) und ii) derart gemischt, dass die Mischung bevorzugt 50 bis 95 Gew.-%, mehr bevorzugt 60 bis 95 Gew.-% und insbesondere bevorzugt 70 bis 90 Gew.-% des Tonminerals vom Typ i) und 5 bis 50 Gew.-%, bevorzugt 5 bis 40 Gew.-% und insbesondere 10 bis 30 Gew.-% des Tonminerals vom Typ ii) enthält.

Wie bereits ausgeführt, wird die Tonmineral-Mischung mit quaternären Ammoniumverbindungen modifiziert. Geeignete quaternäre Ammoniumverbindungen zur Behandlung von Tonmineralien sowie Verfahren zur Behandlung von Tonmineralien mit solchen Verbindungen sind bekannt.
Geeignete quaternäre Ammoniumverbindungen sind bevorzugt ausgewählt aus quaternären Alkylammoniumsalzen der folgenden Formel (I) 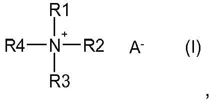 in der
R1 bis R4 für gleiche oder unterschiedliche, geradkettige, vernetzte, gesättigte oder ungesättigte C1-C22-Reste oder für Benzylreste, die mit einer oder mehreren Alkylgruppen substituiert sein können, und
A- für ein Chlorid-, Bromid-, Methylsulfat-, Nitrat-, Hydroxid-, Acetat- und/oder ein Phosphation steht. Bevorzugt steht mindestens einer der Reste R1 bis R4 für eine geradkettige C1-C4-Alkylgruppe, bevorzugt für eine Methylgruppe, oder für einen Benzylrest, der mit einer oder mehreren C1-C4-Alkylgruppen, bevorzugt mit Methylgruppen, substituiert sein kann.
A- steht bevorzugt für ein Chlorid-, Bromid- oder ein Methylsulfation.

Beispiele für besonders bevorzugte quaternäre Ammoniumverbindungen, mit denen die Tonmineral-Mischung modifiziert sein kein, sind dimethyl di(hydrogenated tallow) ammonium chloride, methylbenzyl di(hydrogenated tallow) ammonium chloride, dimethylbenzyl hydrogenated tallow ammonium chloride, dimethyl hydrogenated tallow-2-ethylhexylammonium chloride sowie Mischungen dieser Salze.
Die Tonmineralmischung wird bevorzugt mit 5 bis 80 Milliequivalent (meq.) der quaternären Ammoniumverbindung (pro 100 g der Tonmineralmischung) behandelt.

In einer bevorzugten Ausführungsform werden die mit quaternären Ammoniumverbindungen modifizierten Tonmineral-Mischungen hergestellt, indem Sepiolith und/oder Palygorskit zerkleinert, in Wasser aufgeschlämmt und gefiltert wird, um Sand und andere Verunreinigungen zu entfernen. Das Tonmineral vom Smektit-Typ wird ebenso vorbehandelt. Die Aufschlämmungen der Tonminerale vom Typ i) und ii) werden weiter mit Wasser verdünnt (1 bis 6% Feststoffe) und in eine Mühle mit hoher Scherkraft geleitet (beispielsweise eine Manton-Gaulin Mühle), um eine Homogenisierung zu erreichen. Die Aufschlämmung wird gegebenenfalls mehrere Male durch die Mühle mit hoher Scherkraft geleitet. Ein solches Verfahren ist beispielsweise beschrieben in der US-Patentanmeldung 5,160,454, auf die an dieser Stelle Bezug genommen wird.
Im Anschluss daran können die Aufschlämmungen der Tonminerale vom Typ i) und ii) vermischt, und die quaternäre Ammoniumverbindung kann hinzugegeben werden. Nach der Entwässerung, Trockung und ggfs. Zerkleinerung der Mischungen kann eine erfindungsgemäß geeignete Tonmineral-Mischung c) erhalten werden.

Erfindungsgemäß geeignete, mit quaternären Ammoniumverbindungen modifizierte Tonmineral-Mischungen sind im Handel erhältlich, beispielsweise von der Firma Southern Clay Products unter der Handelsbezeichnung Garamite^{®}. Eine besonders bevorzugte Tonmineralmischung ist Garamite^{®} 7303.

Es wurde gefunden, dass insbesondere soft solid-Produkte mit ausgezeichneten Eingenschaften hergestellt werden können (Synerese < 7,5%; Härte 0,015 - 0,045 mN), wenn der Gehalt des Verdickungsmittels c) >0,5 Gew.-% beträgt, und das Verdickungsmittel b) im Überschuss zum Verdickungsmittel a) eingesetzt wird.
In einer weiteren besonders bevorzugten Ausführungsform beträgt der Gehalt des Verdickungsmittels c) daher mindestens 0,5 Gew.-% - bezogen auf das Gesamtgewicht der Zusammensetzung - und das Gewichtsverhältnis des Verdickungsmittels b) zum Verdickungsmittel a) beträgt 7 : 1 bis 100 : 1, bevorzugt 8 : 1 bis 50 : 1 und insbesondere bevorzugt 9 : 1 bis 25 : 1.

Weitere bevorzugte erfindungsgemäße Zusammensetzungen sind dadurch gekennzeichnet, dass sie, beispielsweise zur weiteren Verbesserung der Konsistenz und/oder der sensorischen Eigenschaften zusätzlich mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind (z.B. Dry FLO PC der Firma AKZO), Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich als erfindungsgemäß bevorzugte Füllstoffe eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).
Bevorzugte erfindungsgemäße Zusammensetzungen sind dadurch gekennzeichnet, dass sie mindestens einen festen, wasserunlöslichen teilchenförmigen Füllstoff in einer Gesamtmenge von 0,1 bis 99 Gew.-%, bevorzugt 0,2 - 90 Gew.-%, besonders bevorzugt 0,3 - 15 Gew.-%, , jeweils bezogen auf die Gesamtzusammensetzung, enthalten.

Weitere bevorzugte erfindungsgemäße Zusammensetzungen sind dadurch gekennzeichnet, dass sie mindestens einen Duftstoff und/oder mindestens ein Parfümöl enthalten.
Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, parat-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Riechstoffe verwendet, die gemeinsam eine ansprechende Duftnote erzeugen.
Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.
Bevorzugte erfindungsgemäße Zusammensetzungen enthalten mindestens einen Duftstoff und/ oder mindestens ein Parfümöl in einer Gesamtmenge von 0,01 - 10 Gew.-%, bevorzugt 0,1 - 5 Gew.-%, besonders bevorzugt 0,5 - 3 Gew.-%, außerordentlich bevorzugt 1 - 2 Gew.-%, jeweils bezogen auf das Gesamtgewicht der erfindungsgemäßen Zusammensetzung.

In einer bevorzugten Ausführungsform werden die erfindungsgemäßen Zusammensetzungen als soft-solid-Antitranspirant konfektioniert. In diesem Fall können sie zusätzlich mindestens einen Deodorant-Wirkstoff enthalten. Bevorzugte derartige Deodorant-Wirkstoffe sind ausgewählt aus Geruchsabsorbern, desodorierend wirkenden Ionenaustauschern, keimhemmenden Substanzen, präbiotisch wirksamen Substanzen sowie Enzyminhibitoren und, besonders bevorzugt, Kombinationen der genannten Deodorant-Wirkstoffe.
Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.
Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.
Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat. Bevorzugte erfindungsgemäße Zusammensetzungen sind dadurch gekennzeichnet, dass der mindestens eine zusätzliche Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.
Weitere bevorzugte erfindungsgemäße Zusammensetzungen sind dadurch gekennzeichnet, dass der mindestens eine zusätzliche Deodorant-Wirkstoff in einer Gesamtmenge von 0,1 - 10 Gew.-%, bevorzugt 0,2 - 7 Gew.-%, besonders bevorzugt 0,3 - 5 Gew.-%, bezogen auf das Gesamtgewicht der Aktivsubstanz in der Gesamtzusammensetzung, enthalten ist.

Antioxidative Stoffe können in erfindungsgemäßen Zusammensetzungen, die als Antitranspirant konfektioniert werden, der oxidativen Zersetzung der Schweißkomponenten entgegenwirken und auf diese Weise die Geruchsentwicklung hemmen. Geeignete Antioxidantien sind Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Lactoferrin), Huminsäuren, Gallensäure, Gallenextrakte, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate, Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte, die diese Antioxidantien enthalten, eingesetzt werden.
Als lipophile, öllösliche Antioxidantien aus dieser Gruppe sind Tocopherol und dessen Derivate, insbesondere Tocopherylacetat, und Carotinoide sowie Butylhydroxytoluol/anisol bevorzugt.
Die Gesamtmenge der Antioxidantien in bevorzugten erfindungsgemäßen Zubereitungen, die als Antitranspirant konfektioniert werden, beträgt 0,001 - 10 Gew.-%, vorzugsweise 0,05 - 5 Gew.-% und insbesondere 0,1 - 2 Gew.-%, bezogen auf die gesamte Zubereitung.
Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind ausgewählt aus den vorstehend genannten Komplexbildnern.

In einer besonders bevorzugten Ausführungsform der Erfindung wird die erfindungsgemäße Zusammensetzung als kosmetisches Produkt konfektioniert, umfassend
i) eine Abgabevorrichtung, umfassend
   - mindestens einen Behälter, der einen Kolben enthält, welcher dem Behälter als Wandung dient und sich durch einen Abgabemechanismus (insbesondere eine Mutter-Spindel-Anordnung) bewegen lässt,
   - mindestens eine Austrittsöffnung, die in Fluidverbindung mit dem besagten Behälter steht, und
ii) eine in dem besagten Behälter befindliche Zusammensetzung des ersten Erfindungsgegenstandes.

Die Zusammensetzung des ersten Erfindungsgegenstandes lässt sich durch Betätigung des Abgabemechanismusses aus der Austrittsöffnung der Abgabevorrichtung ausbringen. Dabei wird der Kolben bewegt und die Zusammensetzung des ersten Erfindungsgegenstandes in Richtung der Austrittsöffnung gedrückt. Durch den dabei entstehenden Druck, wird die besagte Zusammensetzung durch die mindestens eine Austrittsöffnung aus der besagten Abgabevorrichtung hinaus getrieben.

Die erfindungsgemäßen wasserfreien Zusammensetzungen, besonders jene, die als soft solid konfektioniert werden und insbesondere jene, sie als soft solid-Antitranspirantzusammensetzung konfektioniert werden, weisen den Vorteil auf, das sie eine minimale Synerese (< 7,5%) bei optimaler Härte (0,015 bis 0,045 mN) aufweisen.
Gleichzeitig hinterlassen sie auf der Haut ein trockenes, seidiges Gefühl und auf der Kleidung keinerlei Rückstände.

Ein zweiter Gegenstand der vorliegenden Anmeldung ist die Verwendung von Mischungen aus
- mindestens einem Öl, und
- einer Kombination von Verdickungsmitteln, enthaltend
   a) mindestens ein Siliconelastomer,
   b) mindestens einen geradkettigen oder verzweigten, gesättigten oder ungesättigten Alkohol mit mehr als 18 Kohlenstoffatomen, und
   c) mindestens eine mit quaternären Ammoniumverbindungen modifizierte Tonmineral-Mischung,
als Grundlage für kosmetische soft-solid- und/oder Stiftpräparate. Bevorzugte Ausführungsformen der erfindungsgemäßen Verwendung entsprechen den bevorzugten Ausführungsformen des ersten Erfindungsgegenstandes.

Die nachfolgenden Beispiele sollen die Erfindung verdeutlichen, ohne sie hierauf zu beschränken. Alle Mengenangaben beziehen sich, soweit nicht anders vermerkt, auf Gew.-% bezogen auf das Gesamtgewicht der entsprechenden Zusammensetzung.

### Beispiele:

Die variablen Komponenten 1, 2 und 3 wurden in den in der Tabelle angegebenen Mengen (Gew.-%) mit folgenden konstanten Rezepturbestandteilen zu Antitranspirantien vermischt, bis sich verdickte soft solids gebildet hatten:
- 20 Gew.-% AAZG^{®1} 531 D,
- 3,8 Gew.-% Stearylalkohol,
- 0,5 Gew.-% Aerosil^{®2} 300,
- 65,7 Gew.-% Xiameter^{®3} 0245.

| | **Komponente 1 DC^{®4} 9040 Silicone Elastomer Blend** | **Komponente 2 Garamite^{®5} 7303** | **Komponente 3 Performacol^{®6} 425** | **Synerese [%]** | **Härte [mN]** |
|---|---|---|---|---|---|
| **1** | 4,50 | 0,00 | 5,50 | 9,1 | 0,012 |
| **2** | **4,13** | **1,68** | **4,19** | **7,1** | **0,028** |
| **3** | 5,43 | 0,00 | 4,57 | 6,7 | 0,007 |
| **4** | **3,89** | **1,02** | **5,09** | **4,9** | **0,035** |
| **5** | 4,97 | 2,00 | 3,03 | 9,0 | 0,018 |
| **6** | 6,00 | 0,88 | 3,12 | 12,5 | 0,008 |
| **7** | 5,12 | 0,96 | 3,92 | 11,8 | 0,010 |
| **8** | **3,42** | **0,58** | **6,00** | **6,8** | **0,017** |
| **9** | **2,00** | **2,00** | **6,00** | **4,2** | **0,037** |
| **10** | **3,01** | **2,00** | **4,99** | **4,7** | **0,034** |
| **11** | 6,00 | 2,00 | 2,00 | 8,4 | 0,013 |

1 INCI-Bezeichnung: Aluminum Zirconium Octachlorohydrex GLY; 75-82 Gew.-% AS (Firma Summit Research)
2 INCI-Bezeichnung: Silica (Firma Evonik Degussa)
3 INCI-Bezeichnung: Cyclomethicone (Firma Xiameter, Degussa)
4 INCI-Bezeichnung: Cyclomethicone, Dimethicone Crosspolymer; 12-12,75 Gew.-% Elastomergehalt; Dow Corning
5 INCI-Bezeichnung: Quaternium-90 Sepiolite, Quaternium-90 Montmorillonit (Firma Southern Clay Products)
6 INCI-Bezeichnung: C20-40 Alcohols (Firma Baker Petrolite)

Für die Messungen der Synerese wurden 10 g des jeweiligen Produkts in ein Zentrifugenröhrchen gegeben, für 72 Stunden bei 50°C inkubiert und anschließend für 20 Minuten bei 3900 rpm zentrifugiert.

Die abgeschiedenen klaren Ölphasen wurden jeweils abgenommen, gewogen und in Bezug zum Gesamtprodukt gesetzt.

Für die Bestimmung der Härte wurde die Kraft in mN ermittelt, die notwendig ist, um einen 30° Konus 5 mm tief in die jeweilige Produktmatrix eindringen zu lassen. Dieser Test wurde mit dem Texture Analyzer TA.XT plus durchgeführt.

Die Auswertung der Versuchsserien erfolgte mit "Design Expert" und zeigt (vgl. die Zeilen 2, 4 und 8-10 in der Tabelle) dass bestimmte Zusammensetzungen die Zielbedingungen (Synerese: < 7,5%; Härte: 0,015 bis 0,045 mN) aufweisen.

Dies gilt insbesondere für Zusammensetzungen, die >0,5 Gew.-% Garamite^{®5} 7303 enthalten und die ein Gewichtsverhältnis des Performacol^{®6} 425 zum Siliconelastomer (AS in dem Handelsprodukt DC^{®4} 9040 Silicone Elastomer Blend) von mindestens 7 : 1 aufweisen.

## Patentansprüche

1. Wasserfreie Zusammensetzung, enthaltend
- mindestens ein Öl, und
- eine Kombination von Verdickungsmitteln, enthaltend
a) mindestens ein Siliconelastomer,
b) mindestens einen geradkettigen oder verzweigten, gesättigten oder ungesättigten Alkohol mit mehr als 18 Kohlenstoffatomen, und
c) mindestens eine mit quaternären Ammoniumverbindungen modifizierte Tonmineral-Mischung, wobei
- der Gehalt des Verdickungsmittels c) mindestens 0,5 Gew.-% - bezogen auf das Gesamtgewicht der Zusammensetzung - und
- das Gewichtsverhältnis der Verdickungsmittel b) : a) 7 : 1 bis 100 : 1, bevorzugt 8 : 1 bis 50 : 1 und insbesondere bevorzugt 9 : 1 bis 25 : 1 beträgt.

2. Wasserfreie Zusammensetzung nach Anspruch 1, **dadurch gekennzeichnet, dass** sie zusätzlich mindestens einen Antitranspirant-Wirkstoff in einer Menge von 3 bis 35 Gew.-%, bevorzugt von 5 bis 30 Gew.-% und besonders bevorzugt von 10 bis 25 Gew.-% enthält, wobei sich die Mengen auf das Gesamtgewicht der kristallwasserfreien Aktivsubstanz (USP) in der Gesamtzusammensetzung beziehen.

3. Wasserfreie Zusammensetzung nach einem der Ansprüche 1 oder 2, enthaltend einen Antitranspirant-Wirkstoff, ausgewählt aus wasserlöslichen adstringierenden anorganischen und organischen Salzen des Aluminiums, Zirkoniums und/oder des Zinks, bevorzugt aus Aluminium-Zirkonium-tetra- oder -octa-chlorohydex-Glycin-Komplexen.

4. Wasserfreie Zusammensetzung nach einem der Ansprüche 1 bis 3, **dadurch gekennzeichnet, dass** sie - bezogen auf ihr Gesamtgewicht - 20 bis 85 Gew.-%, bevorzugt 30 bis 80 Gew.-%, besonders bevorzugt 40 bis 75 Gew.-% und ganz besonders bevorzugt 50 bis 70 Gew.-% mindestens eines Öls, bevorzugt mindestens eines flüchtigen Öls und insbesondere mindestens eines flüchtigen Silikonöls, enthält.

5. Wasserfreie Zusammensetzung nach einem der Ansprüche 1 bis 4, **dadurch gekennzeichnet, dass** sie - bezogen ihr Gesamtgewicht - 0,05 bis 3 Gew.-%, bevorzugt 0,1 bis 2,75 Gew.-%, besonders bevorzugt 0,15 bis 2,5 Gew.-% und ganz besonders bevorzugt 0,2 bis 2 Gew.-% mindestens eines Siliconelastomers enthält.

6. Wasserfreie Zusammensetzung nach einem der Ansprüche 1 bis 5, **dadurch gekennzeichnet, dass** das Siliconelastomer erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

7. Wasserfreie Zusammensetzung nach einem der Ansprüche 1 bis 6, **dadurch gekennzeichnet, dass** sie - bezogen ihr Gesamtgewicht - 0,5 bis 10 Gew.-%, bevorzugt 1 bis 8 Gew.-%, besonders bevorzugt 1,5 bis 7 Gew.-% und ganz besonders bevorzugt 2 bis 6 Gew.-% mindestens eines geradkettigen oder verzweigten, gesättigten oder ungesättigten Alkohols mit mehr als 18 Kohlenstoffatomen enthält.

8. Wasserfreie Zusammensetzung nach einem der Ansprüche 1 bis 7, **dadurch gekennzeichnet, dass** sie als Komponente b) geradkettige, gesättigte Alkohole mit durchschnittlich 20 bis 40 Kohlenstoffatomen enthält.

9. Wasserfreie Zusammensetzung nach einem der Ansprüche 1 bis 8, **dadurch gekennzeichnet, dass** sie - bezogen ihr Gesamtgewicht - 0,1 bis 5 Gew.-%, bevorzugt 0,2 bis 4 Gew.-%, besonders bevorzugt 0,3 bis 3 Gew.-% und ganz besonders bevorzugt 0,5 bis 2,5 Gew.-% mindestens einer mit quaternären Ammoniumverbindungen modifizierten Tonmineral-Mischung enthält.

10. Wasserfreie Zusammensetzung nach einem der Ansprüche 1 bis 9, **dadurch gekennzeichnet, dass** die Tonmineral-Mischung (Komponente c))
a. mindestens ein Tonmineral, ausgewählt aus Sepiolith und/oder Palygorskit, und
b. mindestens ein Tonmineral vom Smektit-Typ enthält.

11. Wasserfreie Zusammensetzung nach Anspruch 8, **dadurch gekennzeichnet, dass** das Tonmineral vom Smektit-Typ ausgewählt ist aus Hectorit, Montmorillonit, Bentonit, Beidellit, Saponit, Stevensit, Nontronit und/oder Sauconit.

12. Wasserfreie Zusammensetzung nach einem der Ansprüche 10 oder 11, **dadurch gekennzeichnet, dass** die Tonmineral-Mischung (Komponente c)) 50 bis 95 Gew.-%, mehr bevorzugt 60 bis 95 Gew.-% und insbesondere bevorzugt 70 bis 90 Gew.-% des Tonminerals vom Typ i) und 5 bis 50 Gew.-%, bevorzugt 5 bis 40 Gew.-% und insbesondere 10 bis 30 Gew.-% des Tonminerals vom Typ ii) enthält.

13. Wasserfreie Zusammensetzung nach einem der Ansprüche 10 bis 12, **dadurch gekennzeichnet, dass** die Tonmineral-Mischung (i) und ii)) mit dimethyl di(hydrogenated tallow) ammonium chloride, methylbenzyl di(hydrogenated tallow) ammonium chloride, dimethylbenzyl hydrogenated tallow ammonium chloride, dimethyl hydrogenated tallow-2-ethylhexylammonium chloride sowie Mischungen dieser Salze modifiziert wird.

14. Verwendung von Mischungen aus
- mindestens einem Öl, und
- einer Kombination von Verdickungsmitteln, enthaltend
a) mindestens ein Siliconelastomer,
b) mindestens einen geradkettigen oder verzweigten, gesättigten oder ungesättigten Alkohol mit mehr als 18 Kohlenstoffatomen, und
c) mindestens eine mit quaternären Ammoniumverbindungen modifizierte Tonmineral-Mischung, wobei
- der Gehalt des Verdickungsmittels c) mindestens 0,5 Gew.-% - bezogen auf das Gesamtgewicht der Zusammensetzung - und
- das Gewichtsverhältnis der Verdickungsmittel b) : a) 7 : 1 bis 100 : 1, bevorzugt 8 : 1 bis 50 : 1 und insbesondere bevorzugt 9 : 1 bis 25 : 1 beträgt.
als Grundlage für kosmetische soft-solid- und/oder Stiftpräparate.

## Claims

1. A water-free composition, containing:
- at least one oil and
- a combination of thickening agents, containing:
a) at least one silicone elastomer,
b) at least one straight-chain or branched, saturated or unsaturated alcohol having more than 18 carbon atoms, and
c) at least one clay mineral mixture modified with quaternary ammonium compounds, wherein
- the content of the thickening agent c) is at least 0.5 wt.%, based on the total weight of the composition, and
- the weight ratio of the thickening agent b):a) is from 7:1 to 100:1, preferably from 8:1 to 50:1, and particularly preferably from 9:1 to 25:1.

2. The water-free composition according to claim 1, **characterized in that** it additionally contains at least one antiperspirant active ingredient in an amount of from 3 to 35 wt.%, preferably from 5 to 30 wt.%, and more preferably from 10 to 25 wt.%, the amounts relating to the total weight of the water-of-crystallization-free active substance (USP) in the total composition.

3. The water-free composition according to one of claims 1 or 2, containing an antiperspirant active ingredient selected from water-soluble astringent inorganic and organic salts of aluminum, zirconium and/or zinc, preferably from aluminum zirconium tetrachlorohydrex or octachlorohydrex glycine complexes.

4. The water-free composition according to one of claims 1 to 3, **characterized in that** it contains, based on its total weight, from 20 to 85 wt.%, preferably from 30 to 80 wt.%, more preferably from 40 to 75 wt.% and most preferably from 50 to 70 wt.% of at least one oil, preferably at least one volatile oil and in particular at least one volatile silicone oil.

5. The water-free composition according to one of claims 1 to 4, **characterized in that** it contains, based on its total weight, from 0.05 to 3 wt.%, preferably from 0.1 to 2.75 wt.%, more preferably from 0.15 to 2.5 wt.%, and most preferably from 0.2 to 2 wt.% of at least one silicone elastomer.

6. The water-free composition according to one of claims 1 to 5, **characterized in that** the silicone elastomer can be obtained by cross-linking an organopolysiloxane that contains at least 2 C₂ to C₁₀ alkenyl groups having a terminal double bond in each molecule, with an organopolysiloxane that includes at least 2 silicone-bonded hydrogen atoms in each molecule.

7. The water-free composition according to one of claims 1 to 6, **characterized in that** it contains, based on its total weight, from 0.5 to 10 wt.%, preferably from 1 to 8 wt.%, more preferably from 1.5 to 7 wt.%, and most preferably from 2 to 6 wt.% of at least one straight-chain or branched, saturated or unsaturated alcohol having more than 18 carbon atoms.

8. The water-free composition according to one of claims 1 to 7, **characterized in that** it contains straight-chain, saturated alcohols having, on average, 20 to 40 carbon atoms as component b).

9. The water-free composition according to one of claims 1 to 8, **characterized in that** it contains, based on its total weight, from 0.1 to 5 wt.%, preferably from 0.2 to 4 wt.%, more preferably from 0.3 to 3 wt.%, and most preferably from 0.5 to 2.5 wt.% of at least one clay mineral mixture modified with quaternary ammonium compounds.

10. The water-free composition according to one of claims 1 to 9, **characterized in that** the clay mineral mixture (component c)) contains
a. at least one clay mineral selected from sepiolite and/or palygorskite, and
b. at least one clay mineral of the smectite type.

11. The water-free composition according to claim 8, **characterized in that** the clay mineral of the smectite type is selected from hectorite, montmorillonite, bentonite, beidellite, saponite, stevensite, nontronite and/or sauconite.

12. The water-free composition according to one of claims 10 or 11, **characterized in that** the clay-mineral mixture (component c)) contains from 50 to 95 wt.%, more preferably from 60 to 95 wt.%, and particularly preferably from 70 to 90 wt.% of the clay mineral of type i), and from 5 to 50 wt.%, preferably from 5 to 40 wt.%, and in particular from 10 to 30 wt.% of the clay mineral of type ii).

13. The water-free composition according to one of claims 10 to 12, **characterized in that** the clay mineral mixture (i) and (ii) is modified with dimethyl di(hydrogenated tallow) ammonium chloride, methylbenzyl di(hydrogenated tallow) ammonium chloride, dimethylbenzyl hydrogenated tallow ammonium chloride, dimethyl hydrogenated tallow-2-ethylhexylammonium chloride, and mixtures of these salts.

14. The use of mixtures of
- at least one oil and
- a combination of thickening agents, containing
a) at least one silicone elastomer,
b) at least one straight-chain or branched, saturated or unsaturated alcohol having more than 18 carbon atoms, and
c) at least one clay mineral mixture modified with quaternary ammonium compounds, wherein
- the content of the thickening agent c) is at least 0.5 wt.%, based on the total weight of the composition, and
- the weight ratio of the thickening agent b):a) is from 7:1 to 100:1, preferably from 8:1 to 50:1, and particularly preferably from 9:1 to 25:1,
as a basis for cosmetic soft-solid and/or stick preparations.

## Revendications

1. composition anhydre contenant
- au moins une huile, et
- une combinaison d'épaississants contenant
a) au moins un élastomère de silicone,
b) au moins un alcool saturé ou insaturé, linéaire ou ramifié, comportant plus de 18 atomes de carbone, et
c) au moins un mélange de minéraux argileux modifié avec des composés d'ammonium quaternaire,
- la teneur en épaississant c) étant d'au moins 0,5% en poids, par rapport au poids total de la composition, et
- le rapport en poids des épaississants b):a) étant de 7:1 à 100:1, de préférence de 8:1 à 50:1 et de façon particulièrement préférée de 9:1 à 25:1.

2. Composition anhydre selon la revendication 1, **caractérisée en ce qu'**elle contient en plus au moins un principe actif antisudoral dans une quantité de 3 à 35% en poids, de préférence de 5 à 30% et de manière particulièrement préférée de 10 à 25% en poids, les quantités étant rapportées au poids total du principe actif (USP) exempt d'eau cristalline dans la composition totale.

3. Composition anhydre selon l'une des revendications 1 ou 2, contenant un principe actif antisudoral choisi parmi les sels minéraux et organiques astringent hydrosolubles d'aluminium, de zirconium et/ou de zinc, de préférence les complexes tétra- ou octa-chlorohydex-glycine d'aluminium-zirconium.

4. Composition anhydre selon l'une des revendications 1 à 3, **caractérisée en ce qu'**elle contient, par rapport à son poids total, de 20 à 85% en poids, de préférence de 30 à 80% en poids, de manière particulièrement préférée de 40 à 75% en poids et de manière tout particulièrement préférée de 50 à 70% en poids, d'au moins une huile, de préférence d'au moins une huile volatile, et en particulier d'au moins une huile de silicone volatile.

5. Composition anhydre selon l'une des revendications 1 à 4, **caractérisé en ce qu'**elle contient, par rapport à son poids total, de 0,05 à 3% en poids, de préférence de 0,1 à 2,75% en poids, de manière particulièrement préférée de 0,15 à 2,5% en poids et de manière tout particulièrement préférée de 0,2 à 2% en poids, d'au moins un élastomère de silicone.

6. Composition anhydre selon l'une des revendications 1 à 5, **caractérisée en ce que** l'élastomère de silicone peut être obtenu par réticulation d'un organopolysiloxane, qui comporte au moins deux groupes alcényle en C₂ à C₁₀ dont chaque molécule possède une double liaison terminale, avec un organopolysiloxane dont chaque molécule comporte au moins deux atomes d'hydrogène liés au silicium.

7. Composition anhydre selon l'une des revendications 1 à 6, **caractérisée en ce qu'**elle contient, par rapport à son poids total, de 0,5 à 10% en poids, de préférence 1 à 8% en poids, de manière particulièrement préférée de 1,5 à 7% en poids et de manière tout particulièrement préférée de 2 à 6% en poids, d'au moins un alcool saturé ou insaturé, linéaire ou ramifié, comportant plus de 18 atomes de carbone.

8. Composition anhydre selon l'une des revendications 1 à 7, **caractérisée en ce qu'**elle contient comme composant b) des alcools saturés linéaires comportant en moyenne de 20 à 40 atomes de carbone.

9. Composition anhydre selon l'une des revendications 1 à 8, **caractérisée en ce qu'**elle contient, par rapport à son poids total, de 0,1 à 5% en poids, de préférence de 0,2 à 4% en poids, de manière particulièrement préférée de 0,3 à 3% en poids et de manière tout particulièrement préférée de 0,5 à 2,5% en poids d'au moins un mélange de minéraux argileux modifié avec des composés d'ammonium quaternaire.

10. Composition anhydre selon l'une des revendications 1 à 9, **caractérisée en ce que** le mélange de minéraux argileux (composant c)) contient
a. au moins un minéral argileux choisi parmi la sépiolite et/ou la palygorskite, et
b. au moins un minéral argileux de type smectite.

11. Composition anhydre selon la revendication 8, **caractérisée en ce que** le minéral argileux de type smectite est choisi parmi l'hectorite, la montmorillonite, la bentonite, la beidellite, la saponite, la stevensite, la nontronite et/ou la sauconite.

12. Composition anhydre selon l'une des revendications 10 ou 11, **caractérisée en ce que** le mélange de minéraux argileux (composant c)) contient de 50 à 95% en poids, de préférence de 60 à 95% en poids et de manière particulièrement de préférence de 70 à 90% en poids du minéral argileux i) et de 5 à 50% en poids, de préférence de 5 à 40% en poids et en particulier de 10 à 30% du minéral argileux ii).

13. Composition anhydre selon l'une des revendications 10 à 12, **caractérisée en ce que** le mélange de minéraux argileux (i) et ii)) est modifié avec du chlorure de diméthyl-di(suif hydrogéné)ammonium, le chlorure de méthylbenzyl(suif hydrogéné)ammonium, le chlorure de diméthylbenzyl(suif hydrogéné)ammonium, le chlorure de diméthyl(suif hydrogéné)-2-éthylhexylammonium et des mélanges de ces sels.

14. Utilisation de mélanges
- au moins une huile, et
- une combinaison d'épaississants contenant
a) au moins un élastomère de silicone,
b) au moins un alcool saturé ou insaturé, linéaire ou ramifié, comportant plus de 18 atomes de carbone, et
c) au moins un mélange de minéraux argileux modifié avec des composés d'ammonium quaternaire,
- la teneur en épaississant c) étant d'au moins 0,5% en poids, par rapport au poids total de la composition, et
- le rapport en poids des épaississants b):a) étant de 7:1 à 100:1, de préférence de 8:1 à 50:1 et de façon particulièrement préférée de 9:1 à 25:1,
comme base pour des préparations cosmétiques en forme de bâton et/ou des préparations cosmétiques solides molles.
